# EUROPÄISCHE PATENTSCHRIFT

(11) **EP 0 371 168 B1**
(45) Veröffentlichungstag und Bekanntmachung des Hinweises auf die Patenterteilung: **10.08.1994**
(21) Anmeldenummer: 88120069.5
(22) Anmeldetag: 01.12.1988
(51) Int. Cl.: A61N 5/06

(54) **Reflektoranordnung an einem Bräunungsgerät**
Reflector arrangement for a tanning device
Disposition de réflecteurs pour appareil de bronzage

(43) Veröffentlichungstag der Anmeldung: 06.06.1990
(73) Patentinhaber: EVB-Entwicklungs- und Vertriebsgesellschaft für Bräunungsanlagen mbH, D-39104 Magdeburg (DE)
(72) Erfinder: Lang, Gert, D-3000 Hannover 91 (DE)
(74) Vertreter: Ritter und Edler von Fischern, Bernhard,Dipl.-Ing.

(56) Entgegenhaltungen:
- EP-A- 0 190 533
- US-A- 2 165 449
- US-A- 4 469 102

## Beschreibung

Die Erfindung betrifft eine Reflektoranordnung an einem Bestrahlungsgerät, z.B. einem Bräunungsgerät, insbesondere zur Ganzkörperbräunung, mit einer UV-Strahlungsquelle für die Erzeugung von UV-Strahlung.

Ausgelöst durch den starken Zuspruch, den Bräunungsgeräte in der Öffentlichkeit erfahren, sind mittlerweile zahlreiche verschiedene Geräte dieser Art entwickelt worden, die sich hinsichtlich des Aufbaus in einigen Punkten unterscheiden.

Besonders wirksame Bräunungsgeräte mit hoher Bräunungsleistung bestehen, wie beispielsweise aus EP-A-0 190 533 bekannt, aus einer Liege und einer darüber angeordneten Strahlungseinheit mit UV-Strahlungsquelle, Reflektor und UV-Filtern. Die Strahlungseinheit wird dabei von Stützen im Kopf- und Fußbereich der Liege getragen, so daß zwischen der Liege und der Strahlungseinheit der nötige Freiraum für den Benutzer geschaffen wird.

Neben hoher Bräunungsleistung wird in zunehmendem Maß aber auch eine erhöhte Wirtschaftlichkeit der Geräte verlangt. Daher ist auf zahlreichen Wegen versucht worden, die Strahlungsleistung, und damit auch die Bräunungsleistung zu steigern, ohne daß die aufzubringende elektrische Leistung erhöht wird. Von entscheidender Bedeutung ist dabei die Strahlungsleistung auf der Liegefläche bzw. auf der Körperoberfläche des Benutzers.

In der Strahlungseinheit der bekannten Bräunungsgeräte ist die UV-Strahlungsquelle vor einem Reflektor angeordnet, was aus vielen anderen Anwendungen, auch rein beleuchtungstechnischer Art, allgemein bekannt ist. Dennoch geht ein erheblicher Teil der UV-Strahlung aus verschiedenen Gründen verloren und verschlechtert so das Verhältnis zwischen Bräunungsleistung und aufgewendeter Leistung.

Der Erfindung liegt daher die Aufgabe zugrunde, eine Reflektoranordnung an einem Bestrahlungsgerät, insbesondere einem Bräunungsgerät, zu schaffen, die eine weitere Steigerung der Bräunungsleistung bewirkt.

Gelöst wird diese Aufgabe durch ein Bräunungsgerät mit einer Strahlungseinheit, einer Liegeeinheit, die unterhalb der Strahlungseinheit angeordnet ist, und zwei Stützen, die an den beiden Enden der Liegeeinheit angeordnet sind, an dem erfindungsgemäß eine Reflektoranordnung im Bereich der Stützen und im wesentlichen im Bestrahlungsinnenraum des Bestrahlungsgeräts so ausgebildet und angeordnet ist, daß der auf die Innenflächen der Stützen gerichtete Teil und/oder der neben die Stützen gerichtete Teil der Strahlung der Strahlungseinheit zur Liegeeinheit reflektiert wird.

In einer vorteilhaften Ausführungsform besteht die Reflektoranordnung aus ersten und zweiten Reflektoren, die an den Innenflächen der Stützen bzw. neben den Stützen angeordnet sind.

In einer weiteren vorteilhaften Auführungsform besteht die erfindungsgemäße Reflektoranordnung aus Reflektoren, die auf der Innenfläche der Stützen angeordnet sind.

Weitere Ausgestaltungen der Erfindung ergeben sich aus den Unteransprüchen und aus den aufgezeigten Ausführungsbeispielen der Erfindung.

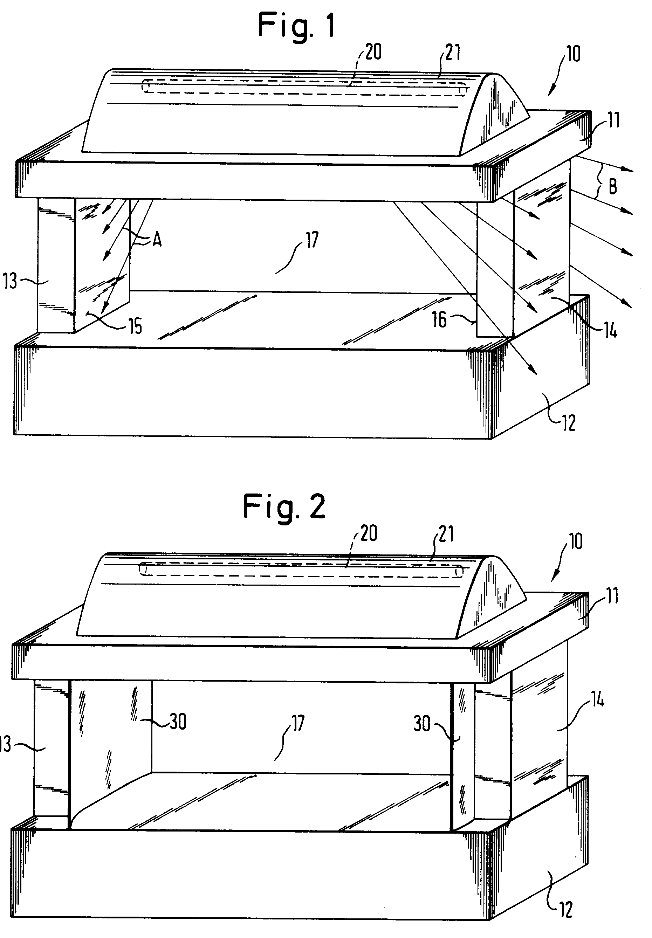

Die Erfindung wird im folgenden anhand der in den Zeichnungen dargestellten Ausführungsbeispielen näher beschrieben. Es zeigt:
- Fig.1: eine perspektivische Ansicht des Aufbaus eines bekannten Bräunungsgeräts
- Fig.2: das bekannte Bräunungsgerät mit einer erfindungsgemäßen Reflektoranordnung,
- Fig.3a - 3c: mehrere Ansichten eines ersten Ausführungsbeispiels eines Bräunungsgeräts, bei dem eine erfindungsgemäße Reflektoranordnung vorgesehen ist,
- Fig.4a - 4c: mehrere Ansichten eines zweiten Ausführungsbeispiels eines Bräunungsgeräts, bei dem eine erfindungsgemäße Reflektoranordnung vorgesehen ist,
- Fig.5a - 5c: mehrere Ansichten eines dritten Ausführungsbeispiels eines Bräunungsgeräts, bei dem eine erfindungsgemäße Reflektoranordnung vorgesehen ist,
Das in Fig.1 dargestellte Bräunungsgerät 10 besteht aus einer Strahlungseinheit 11, einer Liegeeinheit 12 und zwei, am Kopf- und Fußteil der Liegeeinheit angeordneten, Stützen 13 und 14. Auf der Liegeeinheit 12 liegt der Benutzer während der Bestrahlung mit UV-Strahlung, die von einer UV-Strahlungsquelle 20 erzeugt wird und deren Strahlung im wesentlichen von oben auf den Benutzer auftrifft. Die UV-Strahlungsquelle 20 ist in der Strahlungseinheit 11 vor einem Reflektor 21 angeordnet, der die nach oben abgegebene Strahlung in Richtung auf den Benutzer reflektiert.

Unterhalb der Strahlungeinheit 11, oberhalb der Liegeeinheit 12 und zwischen den beiden Stützen 13 und 14 ist der eigentliche Bestrahlungsinnenraum 17 des Bräunungsgeräts festgelegt. Dort hält sich der Benutzer während der Bestrahlung mit der UV-Strahlung auf und dort soll eine ausreichende Strahlungleistung sichergestellt werden.

Wie in Fig.1 weiter gezeigt, trifft ein beachtlicher Teil der UV-Strahlung bei dem bekannten Bräunungsgerät jedoch auf die Innenflächen 15 und 16 Stützen oder geht an den Stützen vorbei in die unmittelbare Umgebung des Bräunungsgeräts. Die auf die Stützen auftreffende Strahlung wird nahezu vollständig absorbiert und bewirkt u.a. eine unerwünschte Erwärmung der Stützen. Der Teil A der UV-Strahlung, der auf die Stützen auftrifft, geht also, ebenso wie der an den Stützen vorbeigehende Teil B der UV-Strahlung verloren, ohne zur beabsichtigten Bräunung beizutragen.

Zur Verdeutlichung der Ursachen für die erheblichen Strahlungsverluste ist in Fig. 1 an der Stütze 13 nur der auftreffende Strahlungsanteil A und an der Stütze 14 nur der an der Stütze vorbeigehende Strahlungsanteil B dargestellt. Es ist jedoch selbstverständlich, daß an beiden Stützen 13 bzw. 14 die Strahlungsverluste aufgrund der beiden oben beschriebenen Ursachen auftreten.

Im folgenden wird anhand Fig.2 die erfindungsgemäße Reflektoranordnung beschrieben.

Um den Strahlungsverlust durch die beiden verlorengehenden Anteile A und B der UV-Strahlung zu vermeiden, sind erfindungsgemäß im Bestrahlungsinnenraum 17 im Bereich der Stützen 13 und 14 Reflektoren 30 vorgesehen. Die Reflektoren bewirken, daß die sonst nutzlosen Anteile A und B der UV-Strahlung wieder in den Bestrahlungsinnenraum 17 zurück abgelenkt werden. Die Strahlungsanteile werden dadurch für den beabsichtigten Zweck, nämlich der Bestrahlung des Benutzers wiederzugeführt. In Fig.2 sind die Reflektoren 30 als leicht gekrümmte oder gebogene Reflektoren ausgestaltet, jedoch können die Reflektoren auch ebene oder auf andere Art vorteilhaft geformte Reflektoren sein. Zur Bündelung der reflektierten Uv-Strahlung sind z.B. parabolische Reflektoren vorstellbar, deren Krümmung lediglich an das gewünschte Bündelungsmaß und die räumlichen Gegebenheiten des Anwendungsfalls anzupassen ist.

Die einstückig ausgebildeten Reflektoren 30 in Fig.2 können auch durch mehrere einzelne Reflektoren ersetzt werden, die an bestimmte zu bestrahlende Abschnitte im Bestrahlungsinnenraum 17 anzupassen sind. Es wird dadurch deutlich, daß durch die Vielzahl der möglichen Reflektoranordnungen gemäß der vorliegenden Erfindung ein hohes Maß an Flexibiltät gewährleistet ist und das die erfindungsgemäße Reflektoranordnung nahezu allen Anforderungen gerecht werden kann.

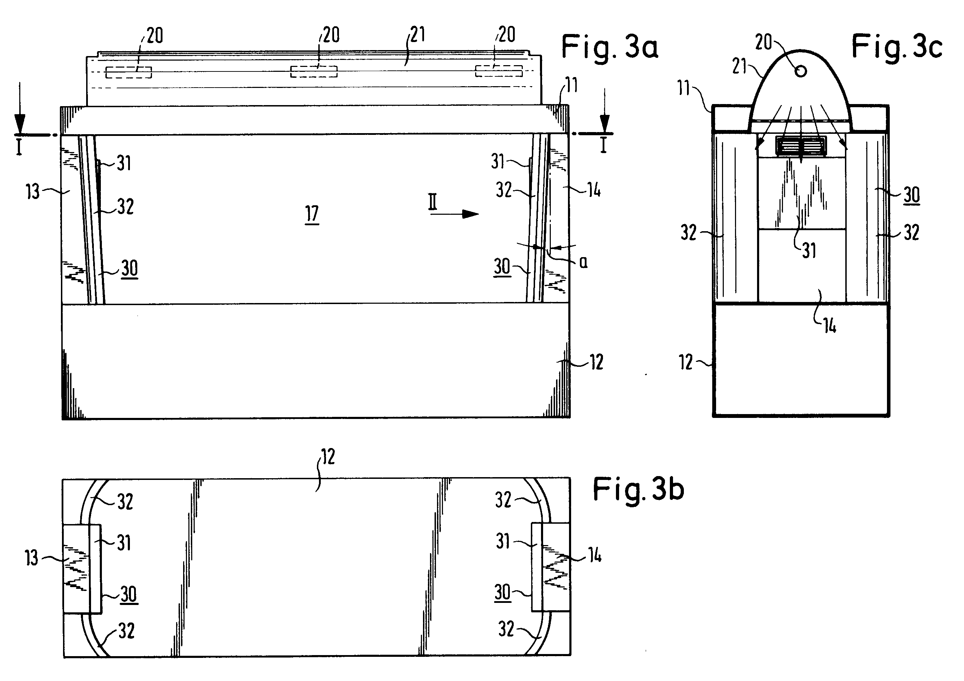

Im folgenden wird anhand der Fig.3a bis 3c ein erstes Ausführungsbeispiel der Erfindung ausführlich beschrieben. Der Aufbau des Bräunungsgeräts entspricht im wesentlichen dem der bekannten Vorrichtung aus Fig.1.

In Fig.3a ist eine Seitenansicht des Bräunungsgeräts dargestellt, das erfindungsgemäß mit einer Reflektoranordnung im Kopf- und Fußbereich ausgestattet ist. In Fig.3b ist eine Querschnittsansicht entlang der Linie I-I in Fig.3a gezeigt, wohingegen in Fig.3c eine Ansicht der Refelektoranordnung an einer der Stützen des Bräunungsgeräts gesehen in Richtung des Pfeils II in Fig.3a dargestellt ist.

Die erfindungsgemäße Reflektoranordnung 30 an den Stützen 13 und 14 des Bräunungsgeräts 10 umfaßt erste Reflektoren 31, die unmittelbar an den Stützen 13 und 14 angeordnet sind, und zweite Reflektoren 32, die auf beiden Seiten neben den Stützen 13 und 14 angeordnet sind.

Die ersten Reflektoren 31 bewirken, daß der Teil A der UV-Strahlung auf den Benutzer auf der Liegeeinheit 12 zurückgeworfen wird. Die Reflektoren 31 sind entweder parallel zur Oberfläche der Stützen auf deren Innenseite angeordnet, wie in Fig.3a gezeigt, oder sind um einen geeigneten Winkel bezogen auf die Senkrechte geneigt. Durch Neigung der Refelektoren 31 kann die refelektierte UV-Strahlung auf die Liegefläche und damit auf den Benutzer ausgerichtet werden.

Die zweiten Reflektoren 32 reflektieren den Teil B der UV-Strahlung in den Bestrahlungsinnenraum 17. Auch die Reflektoren 32 sind entweder parallel zur Oberfläche der Innenseite der Stützen 13 und 14 angeordnet oder sind um einen geeigneten Winkel a bezogen auf die Senkrechte geneigt, wie in Fig.3a dargestellt. Durch die Neigung der Reflektoren 32 kann ebenfalls eine Ausrichtung der reflektierten UV-Strahlung erfolgen, sodaß eine weitere Steigerung der Wirkung der Reflektoren 32 möglich ist.

Wie aus der Darstellung in Fig.3b hervorgeht sind die zweiten Reflektoren 32 um ihre Längsachse gekrümmt, wodurch eine verstärkende Bündelung der reflektieren UV-Strahlung zu erzielen ist. Die Krümmung der Reflektoren 32 ist dabei abhängig vom zu bestrahlen Abschnitt des Bestrahlungsinnenraums 17 und kann zu diesem Zweck an die jeweiligen Anforderungen in weiten Bereichen angepaßt werden. Die Reflektoren 32 können aber auch eben oder um die Querachse gekrümmt ausgebildet sein, wenn es der betreffende Anwendungsfall erfordert.

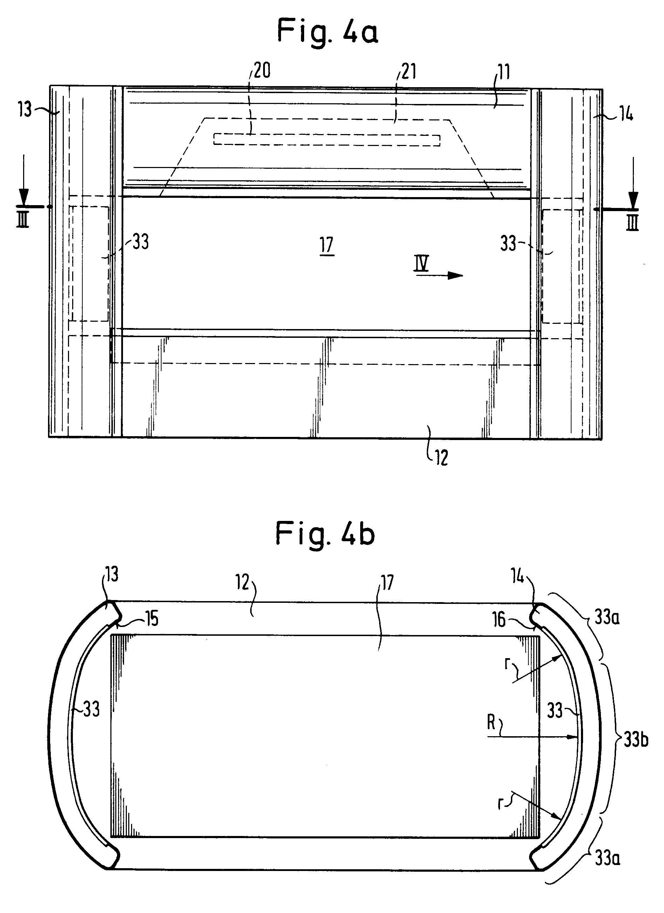

Im folgenden wird anhand der Fig.4a bis 4c ein zweites Ausführungsbeispiel der Erfindung ausführlich beschrieben.

Bei diesem Ausführungsbeispiel sind die beiden Stützen 13 und 14 so ausgebildet, daß die gesamte Breite der Liegeeinheit 12 im Kopf- und Fußbereich abgedeckt wird und somit im wesentlichen nur der unmittelbar auf die Stützen auftreffende Teil A der oben angesprochenen UV-Strahlungsverluste vorhanden ist.

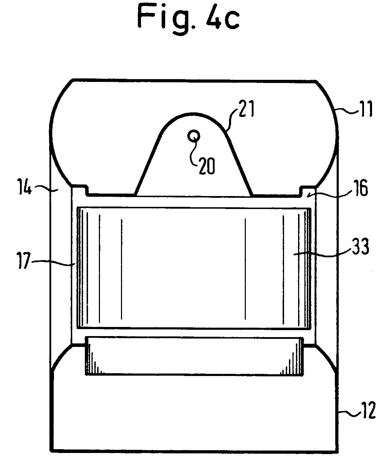

Fig.4a zeigt eine Seitenansicht des Bräunungsgeräts, das erfindungsgemäß mit einer Reflektoranordnung im Kopf- und Fußbereich ausgestattet ist. In Fig.4b ist eine Querschnittsansicht entlang der Linie III-III in Fig.4a gezeigt, wohingegen in Fig.4c eine Ansicht der Refelektoranordnung an einer der Stützen des Bräunungsgeräts gesehen in Richtung des Pfeils IV in Fig.4a, dargestellt ist.

Die erfindungsgemäße Reflektoranordnung an den Stützen 13 und 14 des Bräunungsgeräts 10 besteht in diesem Fall aus einteiligen Reflektoren 33, die unmittelbar an den Stützen 13 und 14 angeordnet sind und sich jeweils nahezu über den gesamten Querschnitt der Innenflächen 15 und 16 der Stützen erstrecken.

Die Reflektoren 33 bewirken, daß der im wesentlichen vorhandene Teil A der UV-Strahlung auf den Benutzer auf der Liegeeinheit 12 zurückgeworfen wird. Die Reflektoren 33 sind entweder parallel zur Längsachse der Stützen auf deren Innenseite angeordnet, wie in Fig.4a gezeigt, oder sind um einen geeigneten Winkel bezogen auf die Senkrechte geneigt. Durch Neigung der Refelektoren 33 kann die refelektierte UV-Strahlung, wie bereits oben erwähnt, auf die Liegefläche und damit auf den Benutzer ausgerichtet werden.

Die beiden Reflektoren 33 sind um eine senkrechte Achse gekrümmt, wie aus Fig.4b ersichtlich ist, und folgen in diesem Ausführungsbeispiel dabei der Form der ebenfalls gebogenen Stützen 13 und 14. Die Krümmung der Reflektoren kann kontinuierlich oder abschnittsweise eben bzw. gekrümmt sein und darüberhinaus in einzelnen Abschnitten unterschiedlich stark ausgeprägt sein. In Fig.4b ist angedeutet, daß die Krümmung in den Randbereichen 33a stärker ist als in den zentralen Bereichen 33b, d.h. daß die Krümmungsradien r in den Randbereichen 33a kleiner sind als die Krümmungsradien R im den zentralen Bereichen 33b. Durch diese Krümmung der Reflektoren 33 wird eine gesteigerte Wirkung der Reflektion erzielt. Jedoch sind bei Stützen mit ebenen Innenseiten auch eben ausgebildete Reflektoren möglich.

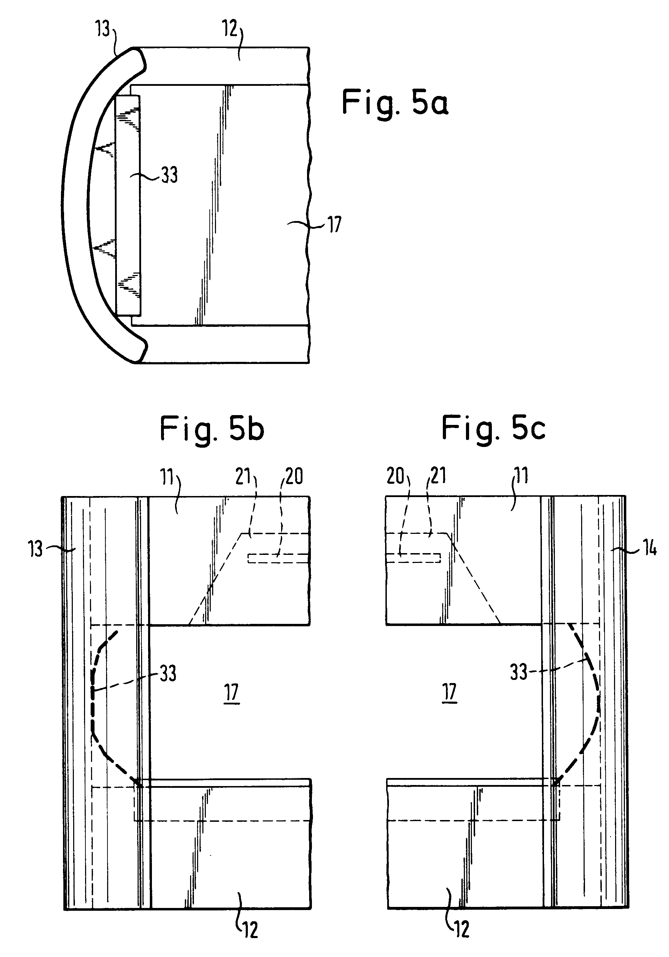

Im folgenden wird anhand der Fig.5a bis 5c ein drittes Ausführungsbeispiel der Erfindung beschrieben.

Das Bräununungsgerät entspricht in diesem Ausführungsbeispiel dem in den Fig.4a bis 4c gezeigten Bräunungsgerät, sodaß dessen Aufbau nicht näher erläuert, sondern auf die vorangegangene Beschreibung an dieser Stelle Bezug genommen wird.

Die erfindungsgemäße Reflektoranordnung an den Stützen des Bräunungsgeräts besteht nun aber aus einteiligen Reflektoren 33, die vor den Stützen 13 bzw. 14 angeordnet sind und sich jeweils nahezu über den gesamten Querschnitt der Innenflächen der Stützen erstrecken.

Die Reflektoren 33 sind gekrümmt, wie aus den Fig.5b und 5c ersichtlich ist. Die Krümmung verläuft in diesem Ausführungsbeispiel quer zur Krümmung der ebenfalls gebogenen Stützen 13 und 14. Die Krümmung der Reflektoren kann kontinuierlich oder abschnittsweise eben bzw. gekrümmt sein und darüberhinaus in einzelnen Abschnitten unterschiedlich stark ausgeprägt sein. In Fig.5b ist gezeigt, wie die Krümmung des Reflektors 33 durch abschnittsweise ebene Bereiche ausgebildet ist. In Fig. 5c ist ein kontinuierlich gekrümmter Reflektor 33 dargestellt.

Die unterschiedlich ausgebildeten Reflektoren können in mehreren Zusammenstellungen an den beiden Stützen des Bräungsgeräts vorgesehen werden. Eine vorteilhafte Ausgestaltung der Refelektoranordnung weist an der Kopfstütze einen kontinuierlich und quer zur Krümmung der Innenfläche der Stütze gekrümmten Reflektor und an der Fußstütze einen abschnittsweise ebenen und ebenfalls quer zur Krümmung der Innenfläche der Stütze gekrümmten Reflektor auf.

Die vorliegende Erfindung wurde an dieser Stelle anhand einer Anwendung an einem Bräunungsgerät beschrieben. Jedoch ist der Einsatz der erfindungsgemäßen Reflektoranordnung nicht auf diese Art eines Bestrahlungsgeräts beschränkt, sondern kann vielmehr auf verschiedene andere Bestrahlungsgeräte mit dem oben beschriebenen Aufbau übertragen und dort eingesetzt werden.

## Patentansprüche

1. Bestrahlungsgerät mit
- einer Strahlungseinheit (11), die eine Strahlungsquelle (20) und einen Reflektor (21) umfaßt,
- einer Liegeeinheit (12), die unterhalb der Strahlungseinheit (11) angeordnet ist, und
- zwei Stützen (13, 14), die an den beiden Enden der Liegeeinheit (12) angeordnet sind,
wodurch ein Bestrahlungsinnenraum (17) festgelegt wird, der von der Strahlungseinheit, der Liegeeinheit und den beiden Stützen bestimmt wird,
**gekennzeichnet** durch
eine Reflektoranordnung (30; 31, 32; 33), die im Bereich der Stützen (13, 14) und im wesentlichen im Bestrahlungsinnenraum (17) vorgesehen sind, zur Reflektion des auf die zum Bestrahlungsinnenraum (17) liegenden Flächen der Stützen (13, 14) gerichteten Teils (A) und/oder des neben die Stützen (13, 14) gerichteten Teils (B) der Strahlung der Strahlungseinheit (11) in den Bestrahlungsinnenraum (17).

2. Bestrahlungsgerät nach Anspruch 1
dadurch **gekennzeichnet**, daß die Reflektoranordnung (30) erste Reflektoren (31), die unmittelbar an den zum Bestrahlungsinnenraum liegenden Flächen (15,16) der Stützen (13,14) angeordnet sind, und zweite Reflektoren (32) umfaßt, die neben den Stützen (13,14) angeordnet sind.

3. Bestrahlungsgerät nach Anspruch 2 dadurch gekennzeichnet, daß die ersten Reflektoren (31) im wesentlichen parallel zur zum Bestrahlungsinnenraum liegenden Fläche (15 bzw. 16) der Stützen angeordnet sind.

4. Bestrahlungsgerät nach Anspruch 2
dadurch **gekennzeichnet**, daß
die ersten Reflektoren (31) bezogen auf die zum Bestrahlungsinnenraum liegende Fläche (15 bzw. 16) der Stützen geneigt sind.

5. Bestrahlungsgerät nach einem der Ansprüche 2 bis 4
dadurch **gekennzeichnet**, daß
die ersten Reflektoren (31) eben sind.

6. Bestrahlungsgerät nach einem der Ansprüche 2 bis 5
dadurch **gekennzeichnet**, daß
die zweiten Reflektoren (32) im wesentlichen parallel zur zum Bestrahlungsinnenraum liegenden Fläche (15 bzw. 16) der Stützen angeordenet sind.

7. Bestrahlungsgerät nach einem der Ansprüche 2 bis 5
dadurch **gekennzeichnet**, daß
die zweiten Reflektoren (32) bezogen auf die zum Bestrahlungsinneraum liegende Fläche (15 bzw. 16) der Stützen geneigt ist.

8. Bestrahlungsgerät nach einem der Ansprüche 2 bis 7
dadurch **gekennzeichnet**, daß
die zweiten Reflektoren (32) eben sind.

9. Bestrahlungsgerät nach einem der Ansprüche 2 bis 7
dadurch **gekennzeichnet**, daß
die zweiten Reflektoren (32) um ihre Längsachse gekrümmt sind.

10. Bestrahlungsgerät nach Anspruch 1
dadurch **gekennzeichnet**, daß
die Reflektoranordnung (30) einteilige Reflektoren (33) umfaßt, die sich nahezu vollständig über die zum Bestrahlungsinnenraum liegenden Flächen (15,16) der Stützen erstrecken.

11. Bestrahlungsgerät nach Anspruch 10
dadurch **gekennzeichnet**, daß
die Reflektoren (33) eben sind.

12. Bestrahlungsgerät nach Anspruch 10
dadurch **gekennzeichnet**, daß
die Reflektoren (33) gekrümmt sind.

13. Bestrahlungsgerät nach Anspruch 12
dadurch **gekennzeichnet**, daß die Krümmung der Reflektoren (33) im wesentlichen parallel zur zum Bestrahlungsinneraum liegenden Fläche (15 bzw. 16) der Stützen verläuft.

14. Bestrahlungsgerät nach Anspruch 12 oder 13
dadurch **gekennzeichnet**, daß
die Reflektoren (33) in einem Randbereich (33a) stärker als in einem zentralen Bereich (33b) gekrümmt sind.

15. Bestrahlungsgerät nach Anspruch 12, 13 oder 14
dadurch **gekennzeichnet**, daß
die Reflektoren (33) kontinuierlich gekrümmt sind.

16. Bestrahlungsgerät nach Anspruch 12, 13 oder 14
dadurch **gekennzeichnet**, daß
die Reflektoren (33) abschnittlsweise eben oder kontinuierlich gekrümmt sind und die ebenen oder kontinuierlich gekrümmten Abschnitte in einem Winkel zueinander angeordnet sind, sodaß jeder der Reflektoren gekrümmt ist.

17. Bestrahlungsgerät nach Anspruch 12
dadurch **gekennzeichnet**, daß die Krümmung der Reflektoren (33) im wesentlichen quer zu der zum Bestrahlungsinneraum liegenden Fläche (15 bzw. 16) der Stützen verläuft.

18. Bestrahlungsgerät nach Anspruch 17
dadurch **gekennzeichnet**, daß
die Reflektoren (33) in einem Randbereich stärker als in einem zentralen Bereich gekrümmt sind.

19. Bestrahlungsgerät nach Anspruch 17 oder 18
dadurch **gekennzeichnet**, daß
die Reflektoren (33) kontinuierlich gekrümmt sind.

20. Bestrahlungsgerät nach Anspruch 17 oder 18
dadurch **gekennzeichnet**, daß
die Reflektoren (33) abschnittsweise eben oder kontinuierlich gekrümmt sind und die ebenen oder kontinuierlich gekrümmten Abschnitte in einem Winkel zueinander angeordnet sind, sodaß jeder der Reflektoren gekrümmt ist.

21. Bestrahlungsgerät nach einem der Ansprüche 10 bis 20
dadurch **gekennzeichnet**, daß
die Reflektoren (33) bezogen auf die zum Bestrahlungsinnenraum liegende Fläche (15 bzw. 16) der Stützen geneigt sind.

## Claims

1. Irradiation appliance with
- a radiation unit (11) which includes a radiation source (20) and a reflector (21),
- a lying unit (12) which is arranged beneath the radiation unit (11), and
- two pillars (13, 14) which are arranged at the two ends of the lying unit (12),
whereby an inner space for irradiation (17) is fixed, which is defined by the radiation unit, the lying unit and the two pillars, characterised by a reflector assembly (30; 31, 32; 33) which is provided in the region of the pillars (13, 14) and essentially in the inner space (17) for irradiation, for reflection into the inner space (17) for irradiation of the portion (A) of radiation of the radiation unit (11) which is directed onto the surfaces of the pillars (13, 14) facing the inner space for irradiation (17) and/or the portion (B) directed adjacent to the pillars (13, 14).

2. Irradiation appliance according to claim 1, characterised in that the reflector assembly (30) includes first reflectors (31) which are arranged directly on the surfaces (15, 16) of the pillars (13, 14) facing the inner space for irradiation, and second reflectors (32) which are arranged adjacent to the pillars (13, 14).

3. Irradiation appliance according to claim 2, characterised in that the first reflectors (31) are arranged essentially parallel to the surface (15 or 16) of the pillars facing the inner space for irradiation.

4. Irradiation appliance according to claim 2, characterised in that the first reflectors (31) are inclined in relation to the surface (15 or 16) of the pillars facing the inner space for irradiation.

5. Irradiation appliance according to any of claims 2 to 4, characterised in that the first reflectors (31) are plane.

6. Irradiation appliance according to any of claims 2 to 5, characterised in that the second reflectors (32) are arranged essentially parallel to the surface (15 or 16) of the pillars facing the inner space for irradiation.

7. Irradiation appliance according to any of claims 2 to 5, characterised in that the second reflectors (32) are inclined in relation to the surface (15 or 16) of the pillars facing the inner space for irradiation.

8. Irradiation appliance according to any of claims 2 to 7, characterised in that the second reflectors (32) are plane.

9. Irradiation appliance according to any of claims 2 to 7, characterised in that the second reflectors (32) are curved about their longitudinal axis.

10. Irradiation appliance according to claim 1, characterised in that the reflector assembly (30) includes one-piece reflectors (33) which extend almost completely over the surfaces (15, 16) of the pillars facing the inner space for irradiation.

11. Irradiation appliance according to claim 10, characterised in that the reflectors (33) are plane.

12. Irradiation appliance according to claim 10, characterised in that the reflectors (33) are curved.

13. Irradiation appliance according to claim 12, characterised in that the curvature of the reflectors (33) extends essentially parallel to the surface (15 or 16) of the pillars facing the inner space for irradiation.

14. Irradiation appliance according to claim 12 or 13, characterised in that the reflectors (33) are curved more in an edge region (33a) than in a central region (33b).

15. Irradiation appliance according to claim 12, 13 or 14, characterised in that the reflectors (33) are continuously curved.

16. Irradiation appliance according to claim 12, 13 or 14, characterised in that the reflectors (33) are plane or continuously curved in sections and the plane or continuously curved sections are arranged at an angle to each other, so that each of the reflectors is curved.

17. Irradiation appliance according to claim 12, characterised in that the curvature of the reflectors (33) extends essentially transversely to the surface (15 or 16) of the pillars facing the inner space for irradiation.

18. Irradiation appliance according to claim 17, characterised in that the reflectors (33) are curved more in an edge region than in a central region.

19. Irradiation appliance according to claim 17 or 18, characterised in that the reflectors (33) are continuously curved.

20. Irradiation appliance according to claim 17 or 18, characterised in that the reflectors (33) are plane or continuously curved in sections and the plane or continuously curved sections are arranged at an angle to each other, so that each of the reflectors is curved.

21. Irradiation appliance according to any of claims 10 to 20, characterised in that the reflectors (33) are inclined in relation to the surface (15 or 16) of the pillars facing the inner space for irradiation.

## Revendications

1. Dispositif d'irradiation, comportant :
- un ensemble d'irradiation (11) comprenant une source de rayonnement (20) et un réflecteur (21),
- un ensemble couchette (10) disposé au-dessous de l'ensemble d'irradiation (11), et
- deux appuis (13, 14) disposés aux deux extrémités de l'ensemble couchette (12),
un espace intérieur d'irradiation (17) étant constitué, déterminé par l'ensemble d'irradiation, l'ensemble couchette et les deux appuis,
caractérisé par
un dispositif réflecteur (30; 31, 32; 33), prévu dans la zone des appuis (13, 14) et sensiblement dans l'espace intérieur d'irradiation (17), pour réfléchir la partie (A) orientée vers les surfaces de l'appui (13, 14) situées dans l'espace intérieur d'irradiation (17) et/ou la partie (B), orientée à côté des appuis (13, 14), du rayonnement émis par l'ensemble d'irradiation (11) dans l'espace intérieur d'irradiation (17).

2. Appareil d'irradiation selon la revendication 1, caractérisé en ce que le dispositif réflecteur (30) comprend des premiers réflecteurs (31) disposés directement sur les surfaces (15, 16), tournées vers l'espace intérieur d'irradiation, des appuis (13, 14) et comprenant des deuxièmes réflecteurs (32) disposés à côté des appuis (13, 14).

3. Appareil d'irradiation selon la revendication 2, caractérisé en ce que les premiers réflecteurs (31) sont disposés sensiblement parallèlement à la surface (15, respectivement 16) des appuis, tournée vers l'espace intérieur d'irridation.

4. Appareil d'irradiation selon la revendication 2 , caractérisé en ce que les premiers réflecteurs (31) sont inclinés par rapport à la surface (15, respectivement 16) tournée vers l'espace intérieur d'irradiation.

5. Appareil d'irradiation selon l'une des revendications 2 à 4, caractérisé en ce que les premiers réflecteurs (31) sont plans.

6. Appareil d'irradiation selon l'une des revendications 2 à 5, caractérisé en ce que les deuxièmes réflecteurs (32) sont disposés sensiblement parallèles à la surface (15, respectivement 16) tournée vers l'espace intérieur d'irradiation, des appuis.

7. Appareil d'irradiation selon l'une des revendications 2 à 5, caractérisé en ce que les deuxièmes réflecteurs (32) sont inclinés par rapport à la surface (15, respectivement 16) tournée vers l'espace intérieur d'irradiation.

8. Appareil d'irradiation selon l'une des revendications 2 à 7, caractérisé en ce que les deuxièmes réflecteurs (32) sont plans.

9. Appareil d'irradiation selon l'une des revendications 2 à 7, caractérisé en ce que les deuxièmes réflecteurs (32) sont incurvés autour de leur axe longitudinal.

10. Appareil d'irradiation selon la revendication 1, caractérisé en ce que le dispositif réflecteur (30) comprend des réflecteurs (31) monoblocs s'étendant à peu près en totalité sur les surfaces (15, 16) tournées vers l'espace intérieur d'irradiation.

11. Appareil d'irradiation selon la revendication 10, caractérisé en ce que les réflecteurs (33) sont plans.

12. Appareil d'irradiation selon la revendication 10, caractérisé en ce que les réflecteurs (33) sont incurvés.

13. Appareil d'irradiation selon la revendication 12, caractérisé en ce que la courbure des réflecteurs (33) s'étend sensiblement parallèle à la surface (15, 16), tournée vers l'espace intérieur d'irradiation, des appuis.

14. Appareil d'irradiation selon la revendication 12 ou 13 caractérisé en ce que les réflecteurs (33) sont incurvés plus fortement dans une zone de bordure (33a) que dans une zone centrale (33b).

15. Appareil d'irradiation selon la revendication 12, 13 ou 14, caractérisé en ce que les réflecteurs (33) ont une courbure continue.

16. Appareil d'irradiation selon la revendication 12, 13 ou 14, caractérisé en ce que les réflecteurs (33), tronçon par tronçon, sont plans ou bien dotés d'une courbure continue et les tronçons plans ou dotés d'une courbure continue sont disposés en faisant les uns par rapport aux autres un angle, de sorte que chacun des réflecteurs est incurvé.

17. Appareil d'irradiation selon la revendication 12, caractérisé en ce que la courbure des réflecteurs (33) s'étend sensiblement transversalement par rapport à la surface (15 respectivement 16), tournée vers l'espace intérieur d'irradiation.

18. Appareil d'irradiation selon la revendication 17, caractérisé en ce que les réflecteurs (33) sont incurvés plus fortement dans une zone de bordure que dans une zone centrale.

19. Appareil d'irradiation selon la revendication 17 ou 18, caractérisé en ce que les réflecteurs (33) sont dotés d'une courbure continue.

20. Appareil d'irradiation selon la revendication 17 ou 18, caractérisé en ce que les réflecteurs (33), tronçon par tronçon, sont plans ou bien dotés d'une courbure continue, et les tronçons plans ou dotés d'une courbure continue sont disposés les uns par rapport aux autres selon un angle, de sorte que chacun des réflecteurs est incurvé.

21. Appareil d'irradiation selon l'une des revendications 10 à 20, caractérisé en ce que les réflecteurs (33 sont inclinés par rapport à la surface (15 respectivement 16), tournée vers l'espace intérieur d'irradiation, des appuis.
